# FASCICULE DE BREVET EUROPEEN

(11) **EP 0 527 063 B1**
(45) Date de publication et mention de la délivrance du brevet: **27.08.2003**
(21) Numéro de dépôt: 92401635.5
(22) Date de dépôt: 12.06.1992
(51) Int. Cl.: C12N 15/00, C12N 15/85, C12N 15/18, C12N 5/10, A01K 67/027, C12N 15/12, C07K 14/00

(54) **Production d'une protéine d'intérêt dans le lait d'un mammifère transgènique**
Herstellung eines Interesse-Proteins in der Milch eines Transgen-Säugetieres
Production of protein of interest in the milk of transgenic mammal

(30) Priorité: 12.06.1991 FR 9107179
(43) Date de publication de la demande: 10.02.1993
(73) Titulaire: INSTITUT NATIONAL DE LA RECHERCHE AGRONOMIQUE, F-75341 Paris Cédex 07 (FR)
(72) Inventeur: Houdebine, Louis-Marie, F-78530 Buc (FR); Devinoy, Eve, F-91190 Gif-sur-Yvette (FR); Thepot, Dominique, F-93193 Livry-Gargan (FR)
(74) Mandataire: Warcoin, Jacques

(56) Documents cités:
- EP-A- 0 264 166
- EP-A- 0 279 582
- WO-A-91/03551
- PROCEEDINGS OF THE NATL. ACADEMY OF SCIENCES USA, vol. 84, mars 1987, Washington, DC (US); A.C. ANDRES et al., pp. 1299-1303
- BIOTECHNOLOGY, vol. 8, février 1990, New York, NY (US); T.A. BÜHLER et al., pp. 140-143
- CELL BIOLOGY INTERNATIONAL REPORTS, vol. 15, no. 2, février 1991; C. PUISSANT et al., pp. 121-129
- NUCLEIC ACIDS RESEARCH, vol. 16, no. 16, 25 août 1988, Arlington, VA (US); E. DEVINOY et al., p. 8180
- DEVINOY E. ET AL: 'Hormone responsive elements within the upstream sequences of the rabbit whey acidic protein (WAP) gene direct chloramphenicol acetyl transferase (CAT) reporter gene expression in transfected rabbit mammary cells' MOLECULAR AND CELLULAR ENDOCRINOLOGY vol. 81, 1991, pages 185 - 193
- BISCHOFF R. ET AL: 'A 17,6 kbp region located upstream of the rabbit WAP gene directs high level expression of a functional human variant in transgenic mouse milk' FEDERATION OF EUROPEAN BIOCHEMICAL SOCIETIES vol. 305, no. 3, Juillet 1992, pages 265 - 268

## Description

La présente invention concerne un procédé de préparation d'une protéine d'intérêt dans le lait d'une femelle de mammifère autre que l'être humain. Elle concerne également les constructions permettant d'obtenir ces animaux, les animaux obtenus ainsi que les cellules contenant les constructions permettant l'expression d'une protéine hétérologue.

Plusieurs voies ont été poursuivies pour obtenir des protéines présentant un intérêt biologique, thérapeutique, ou industriel, et produites naturellement en petites quantités ou sous une forme difficile à purifier.

Des protéines ont ainsi pu être produites grâce à des techniques de recombinaison génétique, par des microorganismes, comme des bactéries ou des levures. Cependant, la plupart des protéines requièrent, après leur synthèse, un stade de maturation consistant en modifications chimiques de certains groupements réactionnels, glycosylation, etc. Les cellules procaryotes ne disposent pas de l'équipement enzymatique adéquat pour effectuer cette maturation, d'où l'obtention de protéines inactives et/ou à fort pouvoir antigénique.

Il est donc préférable de synthétiser ces protéines dans des cellules eucaryotes, qui réaliseront les transformations enzymatiques appropriées. Cependant, la culture à grande échelle de cellules tissulaires pose un certain nombre de difficultés techniques et économiques.

Une autre approche consiste donc à faire produire ces protéines par des cellules in vivo, par l'intermédiaire d'animaux transgéniques. Il est souhaitable que le système utilisé permette la production de protéines en grande quantité, et facilement récupérable. Il est donc intéressant que la protéine recombinante soit produite au niveau de la glande mammaire d'animaux transgéniques, et excrétée dans le lait. Il s'agit en effet d'un fluide biologique aisément collectable, ayant une complexité relativement limitée et une faible activité protéolytique ; en outre, les processus de maturation des protéines recombinantes seront probablement assurés (glycosylation, phosphorylation, clivage, etc.).

On a ainsi réussi à faire synthétiser par la glande mammaire de souris ou de brebis des protéines du lait d'une autre espèce ou des protéines normalement absentes du lait (Réf. 1 à 15). Bühler et al, (Fevrier 1990) Biotechnology, vol 8:140-143 concerne l'utilisation du promoteur de la caséine pour exprimer IL-2 dans le lait des lapins. EP 264 166, NO 91/03551 et Andres et al, (1987) Proc. Natl. Acad. Sci. USA, vol 84:1299-1303 portent sur l'utilisation du promoteur WAP de la souris.

Toutefois, le taux de protéines ainsi produites est extrêmement variable. Il est différent d'un animal transgénique à l'autre, dans la mesure où il est fonction du processus d'intégration du transgène qui est lui-même variable d'un animal à l'autre. La nature des constructions de gène est également essentielle, les éléments régulant l'expression des gènes des protéines du lait pouvant être multiples et situés en divers points de la région promotrice et de la partie transcrite du gène. Ainsi, les promoteurs de la béta-lactoglobuline ovine, de la WAP de rat et de la caséine-béta de rat sont capables de faire synthétiser ces protéines chez des souris transgéniques. Les taux ne sont toutefois systématiquement élevés que dans le cas de la béta-lactoglobuline. De même, le promoteur de la béta-lactoglobuline dirige la synthése de l'alpha₁-antitrypsine humaine qui atteint la valeur de 7 mg/ml de lait dans le lait d'une souris transgénique. Le promoteur de la caséine-alpha_{S1} permet la synthèse de l'urokinase humaine, mais les promoteurs des gènes de la caséine-béta de rat et de la caséine-béta de lapin utilisés jusqu'à ce jour, sont d'une activité limitée. Le promoteur du gène de la WAP de souris dirige la synthèse de plusieurs protéines étrangères (activateur de plasminogène, CD4) qui sont sécrétées dans le lait de souris transgéniques. Les quantités de protéines obtenues avec ce promoteur sont toutefois relativement modestes.

De plus, il arrive que la spécificité du promoteur soit modifiée par son association à un gène étranger. C'est ainsi que Günzburg et al . (Molecular Endocrinology 1991) obtiennent la secrétion d'hormone de croissance à l'aide d'un ADN recombinant sous la dépendance du promoteur de la WAP de souris, chez des animaux transgéniques ; mais l'hormone de croissance est alors également produite dans le cervelet, dans les cellules gliales de Bergman. De tels phénomènes peuvent résulter dans une toxicité et dans la mort prématurée de l'animal.

La présente invention repose sur la mise en évidence de l'intérêt particulier du promoteur du gène de la WAP de lapin. En effet, la WAP de lapin ("whey acidic protein") est une protéine relativement abondante du lait de lapin (15 mg/ml de lait), et le lapin est potentiellement un animal transgénique utilisable à grande échelle. La séquence de l'ADNc codant pour la protéine WAP de lapin est décrite dans Devinoy et al, (1988) Nucleic Acid Research vol 16, page 8180.

La présente invention concerne un procédé de préparation d'une protéine hétérologue d'intérêt sous forme mature ou sous forme fusionnée dans le lait d'une femelle de de mammifère autre que l'être humain, procédé dans lequel :
- on élève ladite femelle et,
- on récupère le lait d'où l'on récupère ladite protéine que l'on sépare si nécessaire,
caractérisé en ce que ladite femelle est un animal transgénique autre qu'un être humain dans le génome duquel a été intégrée une séquence codant pour ladite protéine d'intérêt sous le contrôle d'au moins une séquence figurant parmi les éléments d'expression de la protéine WAP de lapin et se trouvant sur le fragment d'une longueur d'au moins 3 Kb en amont de l'extrémité 3' du promoteur WAP complet.

De préférence, la présente invention concerne un procédé dans lequel la séquence contrôlant l'expression de la protéine d'intérêt comprend en outre des éléments d'expression situés sur le fragment compris entre 3 Kb et 6,3 Kb en amont de l'extrémité 3' du promoteur WAP.

L'obtention d'animaux transgéniques est connue et a été largement décrite avec des constructions voisines dans les documents cités précédemment mais également dans Günzburg et al., Hennighausen et al., Burdon et al., Reddy et al. ainsi que dans le brevet WO 90 05188.

La description détaillée des méthodes de transgénèse ne sera pas rappelée en détail, les documents ci-dessus sont cités dans la présente description par référence expresse.

Par "protéine hétérologue d'intérêt", on entend désigner essentiellement une protéine qui n'est pas naturellement sous le contrôle du promoteur WAP de lapin.

Dans le procédé selon l'invention, la femelle de mammifère autre que l'être humain utilisée est de préférence une lapine mais ces constructions sont efficaces également dans d'autres mammifères, par exemple les souris.

Le lait obtenu contient la protéine d'intérêt qui peut être isolée ou non puis, suivant qu'elle est sous forme mature ou fusionnée, elle peut subir une scission chimique ou enzymatique si nécessaire.

Les constructions d'ADN utilisées sont de préférence introduites par microinjection au niveau de l'oeuf fécondé au stade une cellule jusqu'à 8 cellules puis on sélectionne les animaux répondant aux critères décrits précédemment, c'est-à-dire transgéniques et exprimant ladite protéine dans le lait.

La région promotrice de ce gène WAP greffée au gène rapporteur de la chloramphénicol acétyl transférase (CAT) bactérienne contient les éléments sensibles aux deux hormones lactogènes les plus importantes, la prolactine et les glucocorticoïdes. Ces hormones stimulent de manière intense l'expression du gène CAT lorsque le gène hybride est transfecté dans des cellules épithéliales primaires mammaires de lapine. La réponse aux hormones dépend de la longueur du promoteur utilisé. Le promoteur du gène de la WAP de lapin est donc beaucoup plus efficace que les promoteurs des gènes de la WAP de souris et de rat, utilisés jusqu'à ce jour.

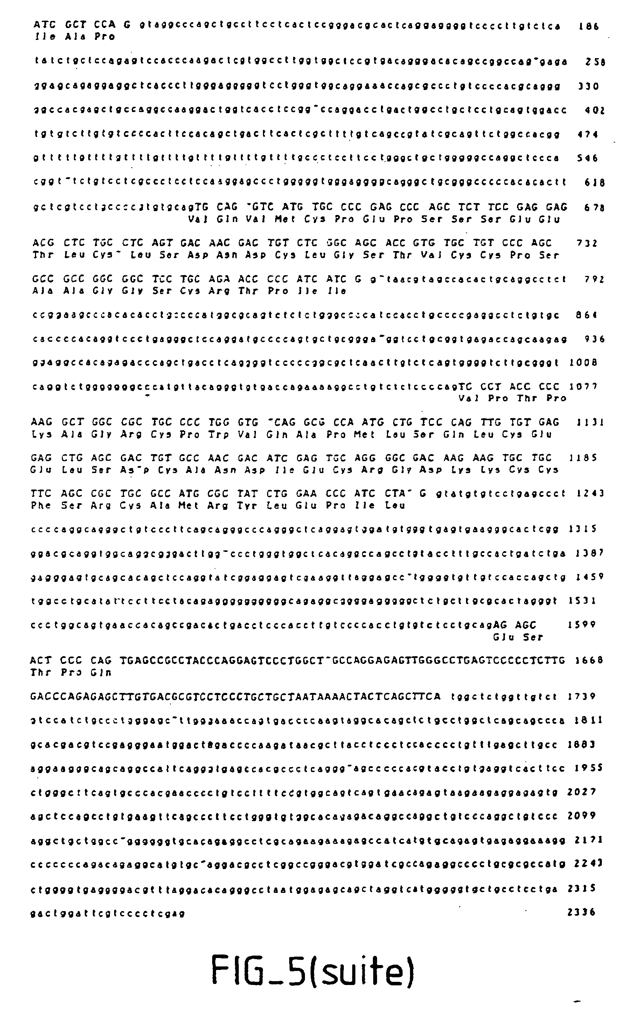

En particulier, dans le procédé selon la présente invention, la séquence codant pour la protéine d'intérêt peut être précédée vers son extrémité 5', d'une séquence correspondant au promoteur du gène WAP de lapin complet, ou d'une séquence équivalente, assurant la fonction de promoteur. Il est même possible, dans ce cas, d'utiliser la totalité du gène WAP et un promoteur WAP dudit gène ou d'une séquence équivalente. La figure 5 annexée à la présente demande représente ledit gène WAP de lapin.

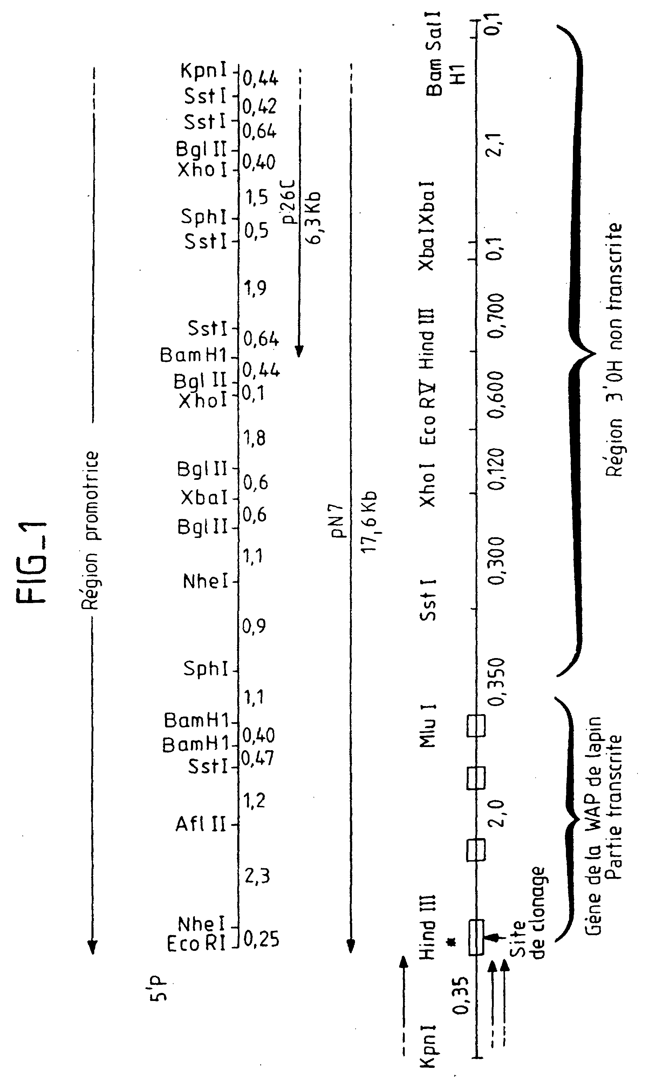

Par "séquence équivalente", on entend désigner de préférence une séquence ayant au moins une longueur de 3 Kb en amont de l'extrémité 3' du promoteur WAP et en particulier comportant les éléments d'expression situés sur le fragment d'une longueur d'au moins 6,3 Kb en amont de l'extrémité 3' du promoteur WAP de lapin complet, notamment située entre les sites HindIII et BamHI (figure 1).

Le promoteur peut comporter une séquence de 17 Kb, comprise entre les sites HindIII et EcoRI, ou une séquence comportant les éléments d'expression situés sur ce fragment. Les éléments essentiels des constructions selon l'invention se trouvent sur le fragment d'une longueur de 17,6 Kb à partir de l'extrémité 3' du promoteur.

Ce long promoteur est susceptible d'apporter des éléments qui favorisent encore l'expression du gène étranger qui lui est lié, ou de rendre son expression plus régulièrement élevée en la rendant plus indépendante du site d'insertion du transgène dans le génome.

Le promoteur court de 6,3 Kb donnera une réponse aux hormones lactogènes pratiquement identique à celle obtenue avec le promoteur long de 17 Kb, et peut diriger la synthèse de protéines dont les gènes lui sont associés dans un vecteur à des taux très élevés.

L'invention concerne également des constructions telles que définies ci-dessus mais dans lesquelles, dans la séquence correspondant au gène de la protéine WAP de lapin, le codon AUG initiateur est délété.

Cette modification peut être obtenue notamment par mutagénèse dirigée.

Lorsque la séquence codant pour la protéine d'intérêt est sous forme fusionnée avec la séquence de tout ou partie du gène WAP, il est possible de supprimer la séquence ATG de cette protéine.

La protéine WAP de lapin pourra ainsi, avec ce type de construction, être exprimée par exemple chez des souris transgéniques.

Dans ce type de construction comprenant le gène de la protéine WAP de lapin, ayant éventuellement perdu le codon AUG initiateur, le gène ou l'ADN_{c} de la protéine d'intérêt peut être placé en différents sites qui pourront conduire à des niveaux et des types de constructions différents, comme cela ressortira des exemples.

Selon un autre de ses aspects, la présente invention fournit un procédé pour récupérer le lait produit par une femelle de mammifère autre que l'être humain et la protéine qu'il contient, caractérisé en ce que, pour récupérer la protéine d'intérêt dans le lait de ladite femelle de mammifère, autre que l'être humain
a) on recueille les glandes mammaires,
b) on laisse incuber les glandes mammaires à une température d'environ 0°C pendant une durée variant de deux heures à 18 heures,
c) on récupère le lait ayant spontanément exsudé des glandes,
d) on isole la protéine d'intérêt à partir du lait et on obtient ladite protéine purifiée.

Ce procédé a été mis au point dans le cadre de la production de protéine hétérologue par un animal transgénique ayant intégré dans son génôme des fragments du gène de la protéine WAP, mais il peut être appliqué à la purification de toute protéine que l'on désire isoler du lait.

Il représente un avantage considérable par rapport aux procédés classiques de traite des mammifères non-ruminants, sur le plan des quantités obtenues, surtout pour des petits animaux comme la souris (24). La composition du lait recueilli est identique à celle du lait produit naturellement. Ce procédé permet en outre un transfert plus aisé des produits obtenus du lieu de production au lieu de purification et de traitement, par transport des glandes mammaires dans de la glace.

Le procédé de préparation de protéine hétérologue de la présente invention, selon l'une quelconque de ses variantes, permet d'obtenir un grand nombre de protéines. Parmi ces protéines, il faut citer :
- les facteurs de croissance,
- les interleukines,
- les facteurs de stimulation,
- les kinases,
- les facteurs de coagulation,
- l'alpha-antitrypsine,
- l'hirudine.
et en particulier :
- l'érythropoïétine,
- le G-CSF,
- l'alpha-antitrypsine,
- l'urokinase,
- le facteur VIII.

On peut citer les constructions suivantes :
- construction WAP-alpha₁-antitrypsine humaine (analogue Arg 358) : le gène entier alpha₁-antitrypsine humaine ayant l'Arg 358 à la place de la Meth 358 (Courtney, Bull. Inst. Pasteur (1988) 86, 85-94) a été fusionné au promoteur long (17,6 Kb) du gène de la WAP de lapin au site Hind III de la séquence 5'P non traduite, sur le modèle des constructions WAP-GH. Plusieurs lignées de souris expriment le gène humain à la concentration de 2 et 5 mg/ml de lait.
- construction WAP-érythropoïétine humaine : le gène entier de l'érythropoïétine humaine (Semenza et al., Proc. Natl. Acad. Sci. USA (1989) 86, 2301-2305) est fusionné au promoteur (6,3 Kb et 17,6 Kb) du gène de la WAP de lapin.
- construction WAP-facteur VIII-deltaII humain : l'ADNc du facteur VIII-deltaII humain (délété de la région B [Meulien et al., Prot. Engin (1988) 2, 301-306]) précédé du premier intron du gène du facteur VIII et dépourvu de sa séquence de polyadénylation, a été introduit à l'intérieur du gène WAP de lapin entier au site Hind III introduit par mutagénèse dirigée et qui précède l'AUG naturel.

Les constructions selon l'invention permettent d'obtenir des résultats tout à fait inattendus, notamment le promoteur de 6,3 Kb de la WAP associé aux gènes de l'hormone de croissance humaine et bovine est capable de diriger la synthèse de ces protéines dans le lait de souris transgéniques à des taux très élevés (1-21 mg/ml).

L'hGH contenue dans le lait des souris transgéniques est structuralement intacte. L'hGH a également conservé son activité biologique (évaluée non par son activité hormone de croissance mais par son activité prolactine). Le test biologique indique que la concentration de l'hormone est de 10 mg/ml, une valeur en accord avec les valeurs trouvées par le test radioimmunologique et par l'électrophorèse.

Le promoteur du gène de la WAP de lapin est donc beaucoup plus efficace que les promoteurs des gènes de la WAP de souris et de rat, utilisés jusqu'à ce jour.

Le tableau 1 suivant donne un résumé des résultats des publications 2 à 15 et met donc en valeur les résultats obtenus avec les constructions selon l'invention, par rapport à ceux publiés dans l'art antérieur.

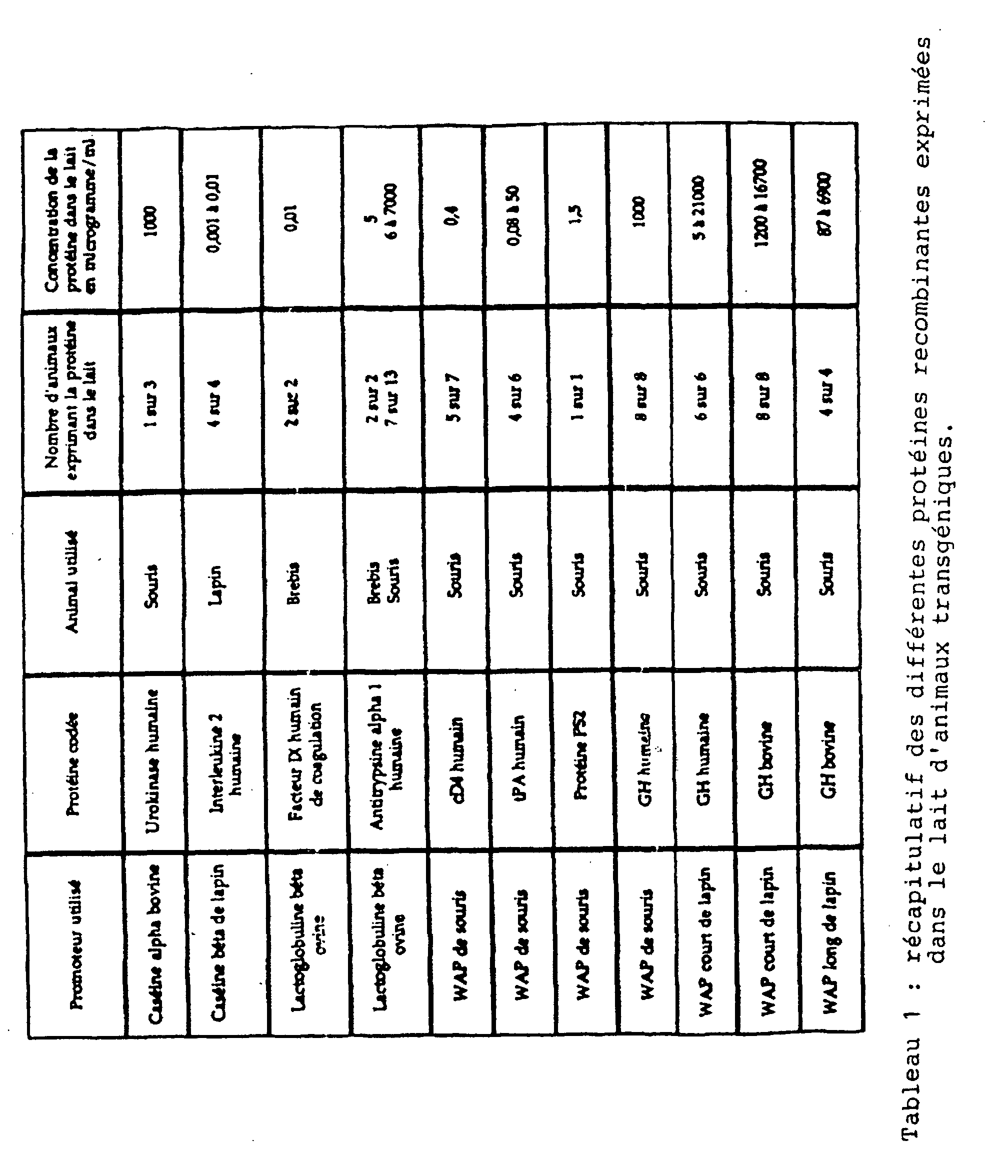

Une des raisons essentielles peut tenir dans le fait que le fragment d'ADN de lapin (6,3 Kb) était beaucoup plus long que son homologue de souris et de rat (2,6 Kb et 0,9 Kb). Des éléments régulateurs essentiels peuvent manquer dans les fragments d'ADN de souris et de rat utilisés.

La présente invention concerne également des constructions permettant d'obtenir des "femelles de mammifère autre que l'être humain" selon l'invention.

La présente invention concerne notamment les séquences d'ADN et les vecteurs permettant la mise en oeuvre du procédé ; en particulier les séquences d'ADN comportant au moins un gène hétérologue codant pour une protéine d'intérêt, sous le contrôle d'au moins une séquence figurant parmi les éléments d'expression de la protéine WAP de lapin et se trouvant sur le fragment d'une longueur d'au moins 3 Kb en amont de l'extrémité 3' du promoteur WAP complet.

La présente invention concerne également des "femelles de mammifère autre que l'être humain" utilisables dans le procédé selon la présente invention, ainsi que des cellules transformées à l'exception des cellules se trouvant in situ dans le corps humain contenant les constructions selon l'invention.

Bien que les animaux en cause puissent être très divers, le lapin est un animal potentiellement utilisable pour obtenir des protéines recombinantes en abondance. Jusqu'à 100 ml de lait peuvent être collectés chaque jour. Ce lait est très riche en protéines (beaucoup plus riche que le lait des ruminants). Il est par ailleurs plus facile et moins onéreux d'obtenir des lapins que des gros animaux transgéniques. Le promoteur du gène de la WAP de lapin a, de plus, toutes les chances de diriger au mieux la synthèse de protéines recombinantes chez le lapin.

D'autres caractéristiques et avantages de la présente invention invention apparaîtront à la lecture des exemples ci-après dans lesquels on se réfèrera aux figures suivantes :
- FIGURE 1: : cartographie du gène de la WAP de lapin.
- FIGURE 2a:: schéma de la construction du plasmide pW₃.
- FIGURE 2b:: polylinker du plasmide p-polyIII-I
- FIGURE 3 :: schéma de la construction du plasmide pJ₄.
- FIGURE 4 :: production d'hormone de croissance humaine et bovine dans des lignées de souris transgéniques abritant les constructions pW₃ et pJ₄.
- FIGURE 5 :: séquence du gène WAP de lapin.
- FIGURE 6 :: schémas des différentes constructions utilisées in vivo.
- FIGURE 7 :: schémas des constructions contenant le gène rapporteur CAT et des longueurs variables du promoteur WAP.
- FIGURE 8 :: efficacité des constructions décrites dans la figure 7.

### EXEMPLE 1 : CONSTRUCTION DU PLASMIDE pW3

On prépare tout d'abord le plasmide p26C.

Le plasmide p26C a été obtenu en introduisant la séquence Bam H₁-Hind III du gène WAP (fragment 6,3 Kb de la Fig. 1) dans le poly linker du vecteur p-poly III-I (entre les sites Bam H₁ et Hind III).

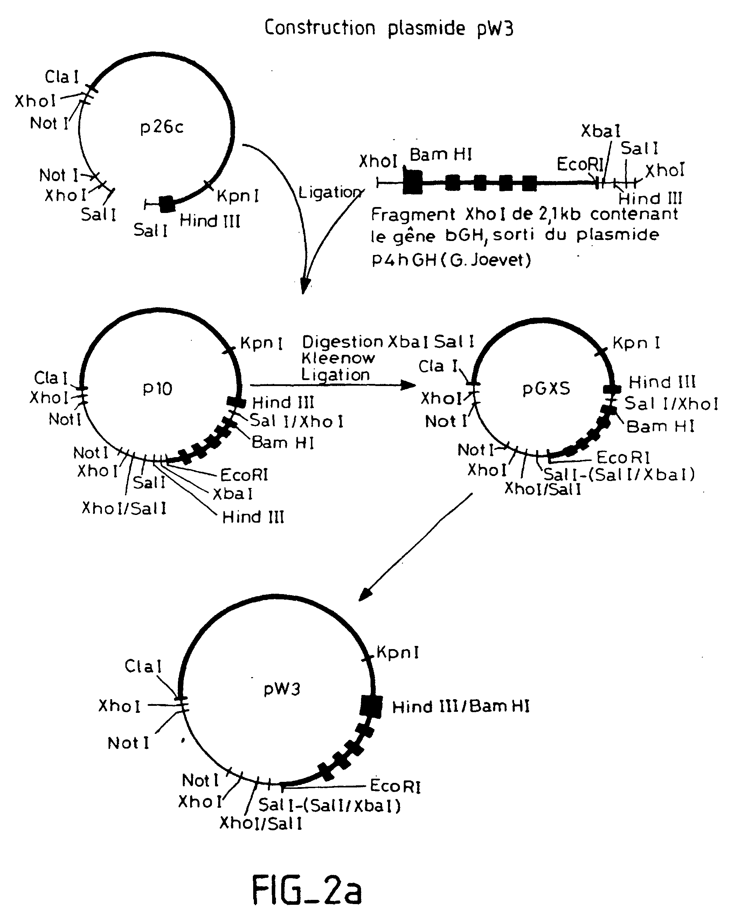

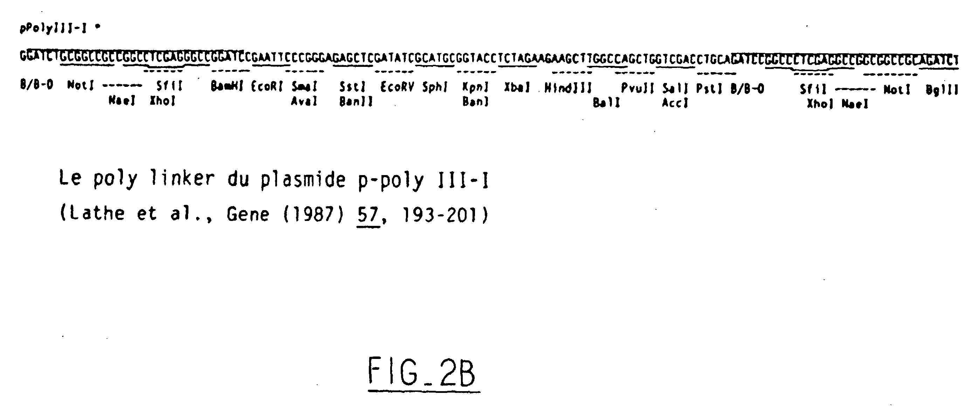

Au cours de ce clonage, le site Bam H₁ a été supprimé et remplacé par le site Cla I qui figure dans le vecteur p26C (Fig.2). Le vecteur p26C est donc un plasmide capable de recevoir un gène étranger placé sous la dépendance du promoteur WAP 6,3 Kb. L'introduction du gène étranger peut se faire par exemple dans le site Sal I du poly linker (Fig.2). Les inserts contenant la totalité du promoteur et des gènes étrangers peuvent être isolés du plasmide après une coupure aux deux sites Not 1 qui sont aux extrémités du poly linker du plasmide p-poly III-I.

Le plasmide pW₃, obtenu à partir du plasmide p26C (selon la figure 2), contient le promoteur du gène de la WAP de lapin (6,3 Kb) et le gène de l'hormone de croissance humaine (hGH). Le fragment utilisé pour obtenir les souris transgéniques est compris entre les deux sites Not1.

Un site Hind III a été introduit dans la séquence de tête du gène (leader) par mutagénèse dirigée pour servir de site de clonage.

### EXEMPLE 2 : CONSTRUCTION DU PLASMIDE pJ4

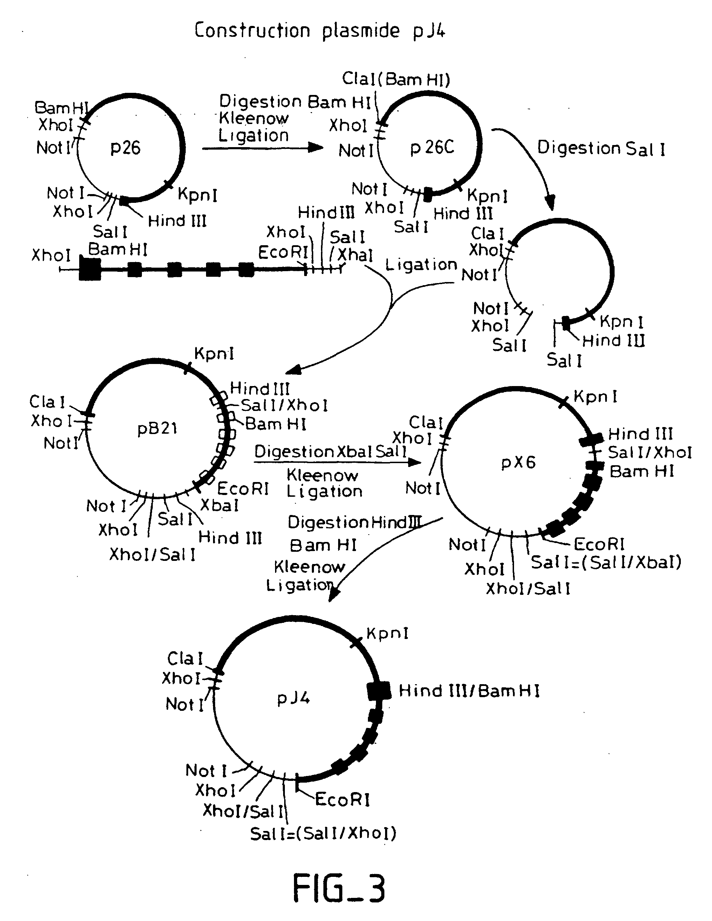

Le plasmide pJ₄, obtenu à partir du plasmide p26 (selon la figure 3), contient le promoteur du gène de la WAP de lapin (6,3 Kb) et le gène de l'hormone de croissance bovine (bGH). Le fragment utilisé pour obtenir les souris transgéniques est compris entre les deux sites Not1.

La souche d'E. Coli contenant le plasmide p26 a été déposée le 12 juin 1991, sous le n° I-1116 à la collection nationale de culture de microorganismes de l'Institut Pasteur, 25 rue du Docteur Roux, 75724 PARIS CEDEX 15.

### EXEMPLE 3 : OBTENTION DE SOURIS TRANSGENIQUES

Les fragments pW₃ et pJ₄ ont été utilisés pour obtenir des animaux transgéniques. Les souris transgéniques ont été obtenues par la technique classique de microinjection (Brinster et al., Proc. Natl. Acad. Sci. USA (1985) 82, 4438-4442). 1-2-pl contenant 500 copies du gène ont été injectés dans le pronucleus mâle d'embryons de souris. Les constructions ont été réalisées dans le vecteur p-poly III-I (Lathe et al., Gene (1987) 57, 193-201). Les fragments Not 1 - Not 1 de ce vecteur contenant les gènes recombinés ont été microinjectés. Les embryons ont ensuite été transférés dans l'oviducte de femelles adoptives hormonalement préparées. Environ 10% des embryons manipulés ont donné naissance à des souriceaux et 2-5 % des embryons manipulés à des souriceaux transgéniques. La présence des transgènes a été révélée par la technique de transfert de Southern à partir de l'ADN extrait des queues des souris. Les concentrations de l'hormone de croissance dans le sang et dans le lait des animaux ont été évaluées à l'aide de tests radioimmunologiques spécifiques.

L'activité biologique de l'hGH a été évaluée en ajoutant du lait au milieu de culture de cellules ou d'explants mammaires de lapin. L'hGH contenue dans le lait a induit l'expression du gène de la caséine-β évaluée par la mesure des ARNm et de la protéine.

### EXEMPLE 4 : PRODUCTION D'HORMONE DE CROISSANCE HUMAINE OU BOVINE DANS LE LAIT DE SOURIS TRANSGENIQUES AYANT INCORPORE LES CONSTRUCTIONS pW3 ET pJ4

Les concentrations d'hormones ont été déterminées par des tests radioimmunologiques spécifiques.

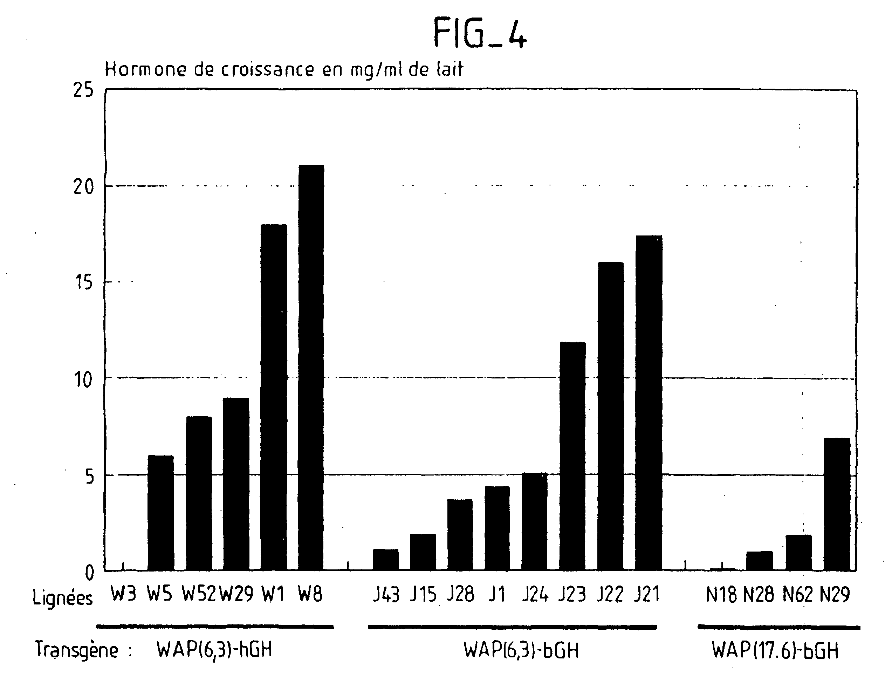

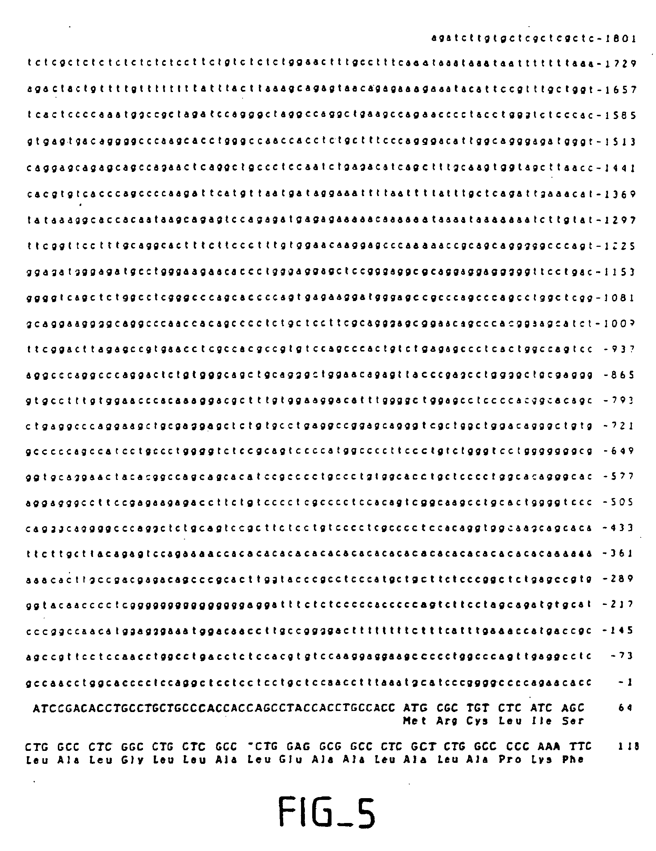

L'identification de l'hGH dans le lait d'une souris transgénique est réalisée de la façon suivante. Le lait de souris est centrifugé à 150.000g pendant une heure pour sédimenter les micelles de caséine. Le surnageant (1 µl par puits) a été récupéré et examiné par une électrophorèse en gel de polyacrylamide, en présence d'hormone de croissance humaine témoin et d'un lait contrôle. Les résultats sont rapportés sur la figure 4.

Les animaux ayant intégré la construction pW₃ donnent des concentrations d'hGH de l'ordre de 10 mg/ml de lait et peuvent atteindre 21 mg/ml.

Les animaux ayant intégré la construction pJ₄ produisent de l'ordre de 5 mg/ml de lait de bGH et jusqu'à 17 mg/ml.

Le procédé selon la présente invention permet de récolter 1,5 ml de lait/glande mammaire de souris (en mettant la glande mammaire dans la glace). 200 souris allaitantes exprimant une protéine étrangère à la concentration de 3 - 5 mg/ml fournissent donc 1 g de la protéine brute.

### EXEMPLE 5 : CONSTRUCTIONS DE GENE

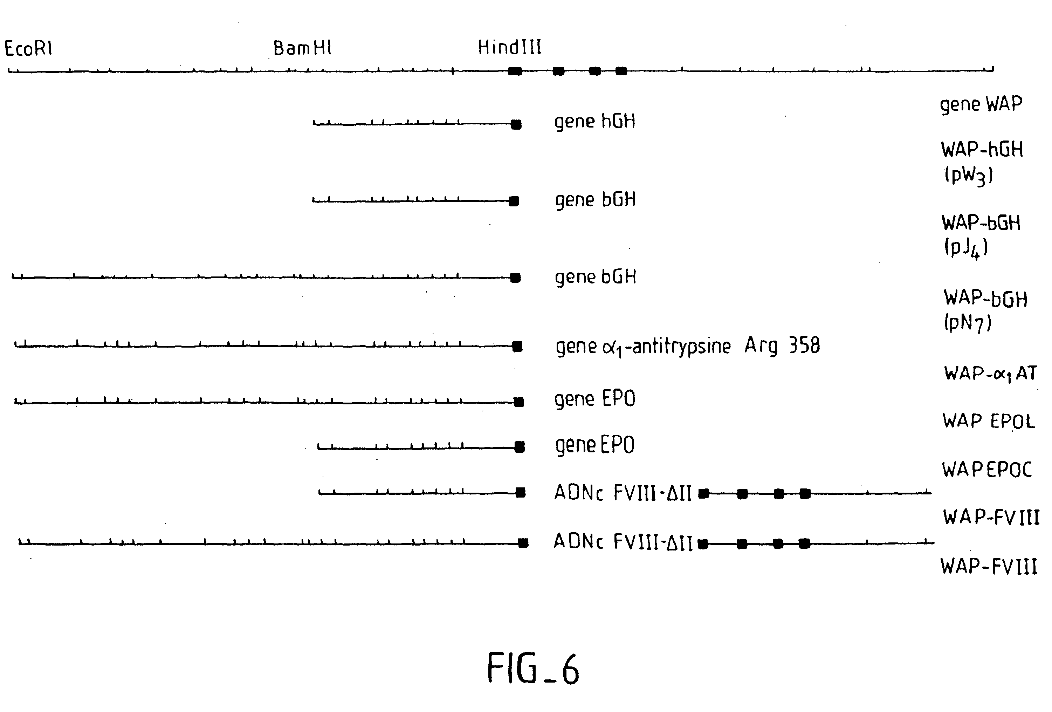

Les constructions des gènes utilisés pour exprimer des protéines recombinantes dans le lait d'animaux transgèniques contiennent dans tous les cas la région régulatrice du gène WAP de lapin : fragment BamHl-HindIII (6,3 Kb) ou fragment EcoRl-HindIII (17,6 Kb). Les plasmides WAP-hGH, WAP bGH, WAPα-AT, et WAP-EPO contiennent respectivement les gènes entiers (séquence de tête, exons, introns et terminateur de transcription) de l'hormone de croissance humaine (hGH), de l'hormone de croissance bovine (bGH), de l'α₁-antitrypsine humaine mutée en Arg358 et d'érythropoeïtine humaine. Dans ces constructions les gènes ont été associés à la région régulatrice du gène WAP au site HindIII. La construction WAP-FVIII-ΔII contient l'ADNc du facteur VIII humain dans sa forme Δ II précédée d'un intron du gène du facteur VIII humain. Cet ensemble intron-ADNc a été introduit dans le site HindIII du gène WAP de lapin entier (figure 6).

### EXEMPLE 6 : IDENTIFICATION DE L'ACTIVITE DE LA REGION REGU-LATRICE DU GENE WAP DE LAPIN IN VITRO

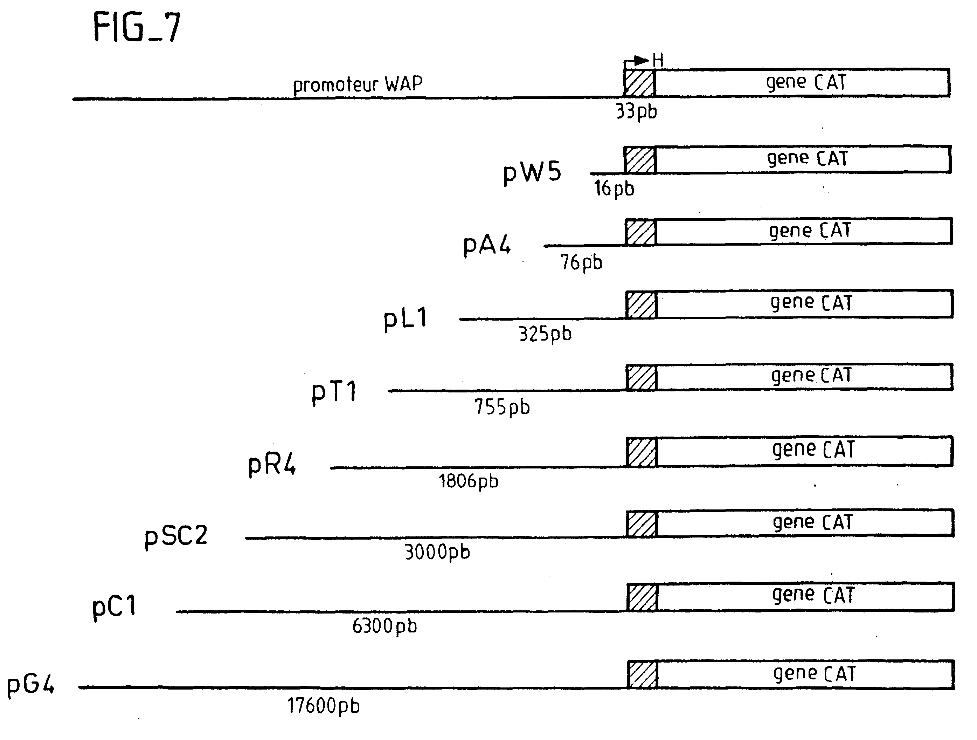

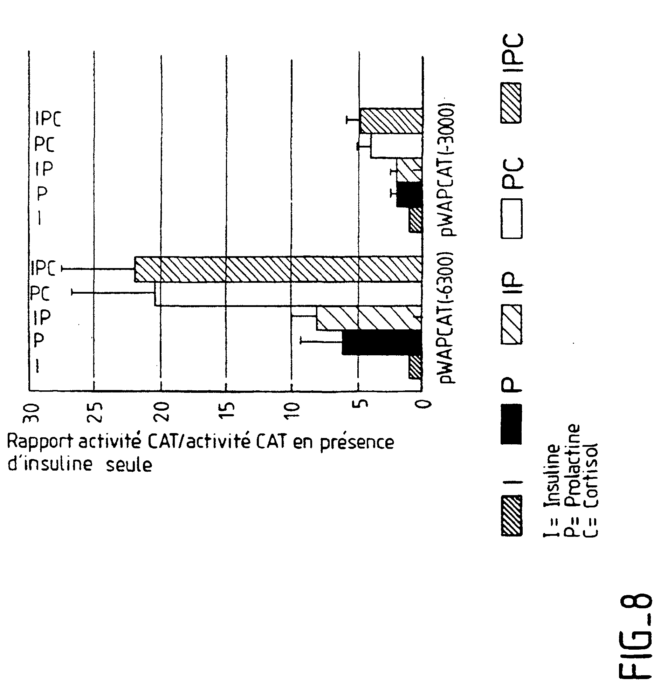

Des longueurs variables de la région située en amont du site d'initiation de la transcription du gène WAP ont été associées à un gène rapporteur (le gène CAT : chloramphenicol acetyl transferase) (figure 7). Ces constructions ont été introduites dans des cellules épithéliales mammaires cultivées sur un gel de collagène I de queue de rat par une transfection à l'aide de lipofectine. Les cellules ont ensuite été maintenues pendant trois jours en présence d'hormones (insuline, cortisol, prolactine). L'enzyme a alors été mesurée dans les extraits cellulaires. Les constructions ne contenant que 1806 pb ou moins de la région régulatrice n'expriment pas le gène CAT. La construction contenant 3000 bp est faiblement active, tandis que les constructions contenant 6300 et 17600 bp sont franchement exprimées en présence des hormones. La prolactine seule exerce un rôle inducteur faible mais significatif sur le gène CAT. L'insuline et surtout le cortisol, inactifs seuls, amplifient l'action de la prolactine. La sensibilité du gène vis-à-vis des hormones est exactement identique à celle du gène WAP endogène des cellules. Les régions -3000-1806 bp et -6300-3000 bp contiennent donc des éléments régulateurs essentiels pour que le gène WAP s'exprime de manière intense (figure 8). Le fragment 17600-6300 bp n'apporte pas de stimulation supplémentaire in vitro ce qui n'exclut pas qu'il puisse avoir une telle action in vitro chez les animaux transgèniques. Ces expériences révèlent pour la première fois l'activité des régions régulatrices du gène WAP in vitro par des transfections des cellules.

### REFERENCES

1. VAN BRUNT J. Biotechnology (1988) 6, 1149-1154
2. SIMONS J.P. et al. Nature (1987) 328, 530-532
3. SIMONS J.P. et al. Biotechnology (1988) 6, 179-183
4. CLARK A.J. Biotechnology (1989) 7, 487-492
5. ARCHIBALD A.L. et al. Proc. Natl. Acad. Sci. USA (1990) 87, 5178-5182
6. HARRIS et al. J. Reprod. Fert. (1990) 88, 707-715
7. GORDON K. et al. Biotechnology (1987) 5, 1183-1187
8. PITTIUS C.W. Proc. Natl. Acad. Sci. USA (1988) 85, 5874-5878
9. PITTIUS C.W. et al. Mol. Endocr. (1988) 2, 1027-1032
10. YU S.H. et al. Mol. Biol. Med. (1989) 6, 255-261
11. LEE K.F. Nucleic Acids Res. (1989) 16, 1027-1040
12. MEADE H. Biotechnology (1990) 8, 443-446
13. BUHLER T.A. Biotechnology (1990) 8, 140-143
14. VILOTTE J.L. Em. J. Biochem. (1989) 186, 43-48
15. BAYNA E.M. et al. Nucleic Acids Res. (1990) 18, 2977-2985
16. LEE K.F. et al. Mol. Cell Biol. (1989) 9, 560-565
17. GRABOWSKI H. et al. (Soumis à publication)
18. DEVINOY E. et al. Nucleic Acids Res. (1988) 16, 11814
19. THEPOT D. et al. Nucleic Acids Res. (1990) 18, 3641
20. GUNZBURG W.H. et al. Molecular Endocrinology (1991). A Mammary-specific Promoter Directs Expression of Growth Hormone not only to the Mammary Gland, but also to Bergman Glia cells in Transgenic Mice.
21. HENNIGHAUSEN L. Protein Expression and Purification (1990) 1, 3-8
22. BURDON T. et al. Expression of a whey acidic protein transgene during mammary development:Evidence for different mechanisms of regulation during pregnancy and lactation
23. REDDY B.V. et al. Human Growth Hormone Expression in Transgenic Mouse Milk, abstract in Transgenes, Development and Disease. p212
24. MASCHIO A. et al. (1991) Biochem. J. 275 454-467

### LEGENDE RELATIVE AUX FIGURES

### FIGURE 6 :

Les constructions utilisées pour obtenir l'expression de protéines recombinantes dans le lait d'animaux transgéniques.

### FIGURE 7 :

Schéma des différentes constructions comportant le gène CAT placé sous la dépendance de longueurs variables du gène de la WAP de lapin.

### FIGURE 8 :

Représentation de la variation de l'activité CAT dans des extraits de cellules épithéliales primaires mammaires de lapin transfectées par différents plasmides.

## Revendications

1. Procédé de préparation d'une protéine hétérologue d'intérêt sous forme mature ou sous forme fusionnée dans le lait d'une femelle de mammifère, autre que l'être humain, procédé dans lequel:
- on élève ladite femelle et,
- on récupère le lait d'où l'on récupère ladite protéine que l'on sépare si nécessaire,
**caractérisé en ce que** ladite femelle est un animal transgénique dans le génome duquel a été intégrée une séquence codant pour ladite protéine d'intérêt sous le contrôle d'au moins une séquence figurant parmi les éléments d'expression de la protéine WAP de lapin et se trouvant sur le fragment d'une longueur d'au moins 3 Kb en amont de l'extrémité 3' du promoteur WAP complet.

2. Procédé selon la revendication 1, **caractérisé en ce que** la séquence codant pour ladite protéine hétérologue d'intérêt est sous le contrôle d'une séquence comportant en outre au moins un élément d'expression situé sur le fragment compris entre 3 Kb et 6,3 Kb en amont de l'extrémité 3' du promoteur WAP de lapin complet.

3. Procédé selon l'une des revendications 1 et 2, **caractérisé en ce que** la séquence codant pour ladite protéine hétérologue d'intérêt est sous le contrôle d'une séquence comprenant tous les éléments d'expression situés sur le fragment d'une longueur de 6,3 Kb en amont de l'extrémité 3' du promoteur WAP de lapin complet.

4. Procédé selon la revendication 3, **caractérisé en ce que** la séquence du promoteur WAP comporte les éléments d'expression situés sur la séquence de 6,3 Kb comprise entre les sites HindIII et BamHI représentés sur la figure 1.

5. Procédé selon l'une des revendications 1 à 4, **caractérisé en ce que** la séquence codant pour ladite protéine hétérologue d'intérêt est sous le contrôle d'une séquence comprenant tous les éléments d'expression situés sur le fragment d'une longueur de 17 Kb, en amont de l'extrémité 3' du promoteur WAP de lapin complet.

6. Procédé selon la revendication 5, **caractérisé en ce que** la séquence du promoteur WAP comporte les éléments d'expression situés sur la séquence de 17 Kb, comprise entre les sites HindIII et EcoRI représentés sur la figure 1.

7. Procédé selon l'une des revendications 1 à 6, **caractérisé en ce que** la séquence codant pour ladite protéine d'intérêt est précédée vers son extrémité 5', d'une séquence correspondant au promoteur du gène WAP de lapin complet, ou d'une séquence équivalente assurant la fonction de promoteur.

8. Procédé selon l'une des revendications 1 à 7, **caractérisé en ce que** ladite protéine d'intérêt est précédée de la totalité du gène WAP et du promoteur WAP dudit gène ou d'une séquence équivalente.

9. Procédé selon la revendication 8, **caractérisé en ce que**, dans la séquence correspondant au gène de la protéine WAP de lapin, le codon AUG initiateur est délété.

10. Procédé selon l'une des revendications 1 à 9, **caractérisé en ce que** dans la séquence correspondant à la séquence codant pour la protéine hétérologue, le codon AUG initiateur est délété.

11. Procédé selon l'une des revendications 1 à 10, **caractérisé en ce que** pour récupérer la protéine d'intérêt dans le lait de ladite femelle de mammifère,
a) on recueille les glandes mammaires,
b) on laisse incuber les glandes mammaires à une température d'environ 0°C pendant une durée variant de deux heures à 18 heures,
c) on récupère le lait ayant spontanément exsudé des glandes,
d) on isole la protéine d'intérêt à partir du lait et on obtient ladite protéine purifiée.

12. Procédé selon l'une des revendications 1 à 11, **caractérisé en ce que** la séquence codant pour la protéine hétérologue est la séquence codant pour l'hormone de croissance humaine.

13. Procédé selon l'une des revendications 1 à 12, **caractérisé en ce que** la protéine hétérologue est choisie parmi :
- les facteurs de croissance,
- les interleukines,
- les facteurs de stimulation,
- les kinases,
- les facteurs de coagulation,
- l'alpha-antitrypsinc,
- l'hirudine.

14. Procédé selon la revendication 13, **caractérisé en ce que** la protéine est choisie parmi :
- l'érythropoïétine,
- le G-CSF,
- l'alpha-antitrypsine,
- l'urokinase
- le facteur VIII.

15. Procédé selon la revendication 14, **caractérisé en ce que** la séquence codant pour la protéine d'intérêt, est constituée par l'ADNc du facteur VIII- deltall humain, précédé du premier intron du gène du facteur VIII.

16. Cellule eucaryote à l'exception des cellules se trouvant in situ dans le corps humain, **caractérisée en ce qu'**elle contient dans son génome une séquence codant pour la protéine d'intérêt hétérologue, sous le contrôle du promoteur WAP de lapin ou d'une séquence équivalente assurant la fonction de promoteur.

17. Cellule eucaryote selon la revendication 16, **caractérisée en ce qu'**il s'agit d'une cellule épithéliale primaire mammaire d'un mammifère femelle.

18. " Femelle de mamnifère transgénique autre que l'être humain", **caractérisée en ce qu'**elle renferme des cellules selon l'une des revendications 16 et 17.

19. Séquence d'ADN, **caractérisée en ce qu'**elle comporte au moins un gène hétérologue codant pour une protéine d'intérêt, sous le contrôle d'au moins une séquence figurant parmi les éléments d'expression de la protéine WAP de lapin et se trouvant sur le fragment d'une longueur d'au moins 3 Kb en amont de l'extrémité 3' du promoteur WAP complet.

## Patentansprüche

1. Verfahren zur Herstellung eines interessierenden heterologen Proteins in reifer Form oder in fusionierter Form in der Milch eines Säugetierweibchens, außer dem Menschen, wobei man in dem Verfahren:
- das Weibchen aufzieht und
- die Milch gewinnt, aus der man das Protein gewinnt, welches man abtrennt, falls erforderlich,
**dadurch gekennzeichnet, dass** das Weibchen ein transgenes Tier ist, in dessen Genom eine Sequenz integriert ist, die für das interessierende Gen unter der Kontrolle mindestens einer Sequenz kodiert, die sich unter den Expressionselementen des WAP-Proteins des Kaninchens befindet und sich auf dem Fragment mit einer Länge von mindestens 3 kb stromaufwärts vom 3'-Ende des vollständigen WAP-Promotors befindet.

2. Verfahren nach Anspruch 1, **dadurch gekennzeichnet, dass** die Sequenz, die für das interessierende heterologe Protein kodiert, unter der Kontrolle einer Sequenz ist, welche darüber hinaus mindestens ein Expressionselement umfasst, das auf dem Fragment zwischen 3 kb und 6,3 kb einschließlich stromaufwärts vom 3'-Ende des vollständigen WAP-Promotors des Kaninchens liegt.

3. Verfahren nach einem der Ansprüche 1 und 2, **dadurch gekennzeichnet, dass** die Sequenz, die für das interessierende heterologe Protein kodiert, unter der Kontrolle einer Sequenz ist, die alle Expressionselemente umfasst, die auf dem Fragment mit einer Länge von 6,3 kb stromaufwärts vom 3'-Ende des vollständigen WAP-Promotors des Kaninchens liegen.

4. Verfahren nach Anspruch 3, **dadurch gekennzeichnet, dass** die Sequenz des WAP-Promotors die Expressionselemente umfasst, die auf der Sequenz mit 6,3 kb liegen, welche zwischen den Hindlll- und BamHI-Stellen eingeschlossen ist, die in Figur 1 dargestellt sind.

5. Verfahren nach einem der Ansprüche 1 bis 4, **dadurch gekennzeichnet, dass** die Sequenz, die für das interessierende heterologe Protein kodiert, unter der Kontrolle einer Sequenz ist, die alle Expressionselemente umfasst, die auf dem Fragment mit einer Länge von 17 kb stromaufwärts vom 3'-Ende des vollständigen WAP-Promotors des Kaninchens liegen.

6. Verfahren nach Anspruch 5, **dadurch gekennzeichnet, dass** die Sequenz des WAP-Promotors die Expressionselemente umfasst, die auf der Sequenz mit 17 kb liegen, welche zwischen den Hindlll- und EcoRI-Stellen eingeschlossen ist, die in Figur 1 dargestellt sind.

7. Verfahren nach einem der Ansprüche 1 bis 6, **dadurch gekennzeichnet, dass** der Sequenz, die für das interessierende Protein kodiert, gegen ihr 5'-Ende eine Sequenz, die dem Promotor des vollständigen WAP-Gens des Kaninchens entspricht, oder eine äquivalente Sequenz vorangeht, welche für die Funktion des Promotors sorgt.

8. Verfahren nach einem der Ansprüche 1 bis 7, **dadurch gekennzeichnet, dass** dem interessierenden Protein das gesamte WAP-Gen und der gesamte WAP-Promotor des Gens oder eine äquivalente Sequenz vorangeht.

9. Verfahren nach Anspruch 8, **dadurch gekennzeichnet, dass** in der Sequenz, welche dem Gen des WAP-Proteins des Kaninchens entspricht, das Initiatorcodon AUG deletiert ist.

10. Verfahren nach einem der Ansprüche 1 bis 9, **dadurch gekennzeichnet, dass** in der Sequenz, die der Sequenz entspricht, welche für das heterologe Protein kodiert, das Initiatorcodon AUG deletiert ist.

11. Verfahren nach einem der Ansprüche 1 bis 10, **dadurch gekennzeichnet, dass** man, um das interessierende Protein in der Milch des Säugetierweibchens zu gewinnen,
a) die Milchdrüsen erntet,
b) die Milchdrüsen bei einer Temperatur von etwa 0°C über eine Dauer, die zwischen 2 Stunden und 18 Stunden variiert, inkubieren lässt,
c) die Milch gewinnt, welche spontan aus den Drüsen ausgeschwitzt wird,
d) das interessierende Protein ausgehend von der Milch isoliert und das gereinigte Protein erhält.

12. Verfahren nach einem der Ansprüche 1 bis 11, **dadurch gekennzeichnet, dass** die Sequenz, die für das heterologe Protein kodiert, die Sequenz ist, die für das menschliche Wachstumshormon kodiert.

13. Verfahren nach einem der Ansprüche 1 bis 12, **dadurch gekennzeichnet, dass** das heterologe Protein ausgewählt ist aus:
- Wachstumsfaktoren,
- Interleukinen,
- Stimulationsfaktoren,
- Kinasen,
- Koagulationsfaktoren,
- alpha-Antitrypsin,
- Hirudin.

14. Verfahren nach Anspruch 13, **dadurch gekennzeichnet, dass** das Protein ausgewählt ist aus:
- Erythropoietin,
- G-CSF,
- alpha-Antitrypsin,
- Urokinase,
- dem Faktor VIII.

15. Verfahren nach Anspruch 14, **dadurch gekennzeichnet, dass** die Sequenz, die für das interessierende Protein kodiert, aus der cDNA des menschlichen Faktors VIII-delta II zusammengesetzt ist, der das erste Intron des Gens des Faktors VIII vorangeht.

16. Eukaryontische Zelle, mit Ausnahme von Zellen, die sich in situ im menschlichen Körper finden, **dadurch gekennzeichnet, dass** sie in ihrem Genom eine Sequenz enthält, die für das interessierende heterologe Protein unter der Kontrolle des WAP-Promotors des Kaninchens oder einer äquivalenten Sequenz kodiert, welche für die Funktion des Promotors sorgt.

17. Eukaryontische Zelle nach Anspruch 16, **dadurch gekennzeichnet, dass** es sich um eine primäre Brustepithelzelle eines weiblichen Säugetiers handelt.

18. Transgenes Säugetierweibchen, außer dem Menschen, **dadurch gekennzeichnet, dass** es Zellen nach einem der Ansprüche 16 und 17 einschließt.

19. DNA-Sequenz, **dadurch gekennzeichnet, dass** sie mindestens ein heterologes Gen umfasst, das für ein interessierendes Protein unter der Kontrolle mindestens einer Sequenz kodiert, die sich unter den Expressionselementen des WAP-Proteins des Kaninchens befindet und sich auf dem Fragment mit einer Länge von mindestens 3 kb stromaufwärts vom 3'-Ende des vollständigen WAP-Promotors befindet.

## Claims

1. Process for the preparation of a heterologous protein of interest in mature form or in fused form in the milk of a mammalian female other than a human being, in which process:
- the said female is bred and,
- the milk is recovered and the said protein is recovered from it and is separated if necessary,
**characterized in that** the said female is a transgenic animal in the genome of which has been integrated a sequence encoding the said protein of interest under the control of at least one sequence present among the elements for expression of the rabbit WAP protein and situated on the fragment having a length of at least 3 Kb upstream from the 3' end of the complete WAP promoter.

2. Process according to Claim 1, **characterized in that** the sequence encoding the said heterologous protein of interest is under the control of a sequence comprising, in addition, at least one expression element situated on the fragment between 3 Kb and 6.3 Kb upstream from the 3' end of the complete rabbit WAP promoter.

3. Process according to either of Claims 1 and 2, **characterized in that** the sequence encoding the said heterologous protein of interest is under the control of a sequence comprising all the expression elements situated on the fragment with a length of 6.3 Kb upstream from the 3' end of the complete rabbit WAP promoter.

4. Process according to Claim 3, **characterized in that** the sequence of the WAP promoter comprises the expression elements situated on the 6.3 Kb sequence between the HindIII and BamHI sites represented in Figure 1.

5. Process according to one of Claims 1 to 4, **characterized in that** the sequence encoding the said heterologous protein of interest is under the control of a sequence comprising all the expression elements situated on the fragment with a length of 17 Kb, upstream from the 3' end of the complete rabbit WAP promoter.

6. Process according to Claim 5, **characterized in that** the sequence of the WAP promoter comprises the expression elements situated on the 17 Kb sequence between the HindIII and EcoRI sites represented in Figure 1.

7. Process according to one of Claims 1 to 6, **characterized in that** the sequence encoding the said protein of interest is preceded towards its 5' end, by a sequence corresponding to the promoter of the complete rabbit WAP gene, or by an equivalent sequence ensuring the promoter function.

8. Process according to Claims 1 to 7, **characterized in that** the said protein of interest is preceded by the entire WAP gene and by the WAP promoter of the said gene or by an equivalent sequence.

9. Process according to Claim 8, **characterized in that**, in the sequence corresponding to the rabbit WAP protein-gene, the initiator AUG codon is deleted.

10. Process according to one of Claims 1 to 9, **characterized in that**, in the sequence corresponding to the sequence encoding the heterologous protein, the initiator AUG codon is deleted.

11. Process according to one of Claims 1 to 10, **characterized in that**, in order to recover the protein of interest from the milk of the said mammalian female,
a) the mammary glands are recovered,
b) the mammary glands are incubated at a temperature of about 0°C for a period ranging from two hours to 18 hours,
c) the milk having spontaneously exuded from the glands is recovered,
d) the protein of interest is isolated from the milk and the said purified protein is obtained.

12. Process according to one of Claims 1 to 11, **characterized in that** the sequence encoding the heterologous protein is the sequence encoding human growth hormone.

13. Process according to one of Claims 1 to 12, **characterized in that** the heterologous protein is chosen from:
- growth factors,
- interleukins,
- stimulating factors,
- kinases,
- coagulation factors,
- alpha-antitrypsin,
- hirudin.

14. Process according to Claim 13, **characterized in that** the protein is chosen from:
- erythropoietin,
- G-CSF,
- alpha-antitrypsin,
- urokinase,
- factor VIII.

15. Process according to Claim 14, **characterized in that** the sequence encoding the protein of interest consists of the cDNA of human factor VIII-deltaII preceded by the first intron of the factor VIII gene.

16. Eukaryotic cell with the exception of cells situated in situ in the human body, **characterized in that** it contains in its genome a sequence encoding the heterologous protein of interest, under the control of the rabbit WAP promoter or of an equivalent sequence ensuring the promoter function..

17. Eukaryotic cell according to Claim 16, **characterized in that** it is a mammary primary epithelial cell of a female mammalian.

18. Transgenic mammalian female other than a human being, **characterized in that** it contains cells according to either of Claims 16 and 17.

19. DNA sequence, **characterized in that** it comprises at least one heterologous gene encoding a protein of interest, under the control of at least one sequence present among the elements for expression of the rabbit WAP protein and located on the fragment having a length of at least 3 Kb upstream 3' end of the complete WAP promoter.
